# EUROPÄISCHE PATENTSCHRIFT

(11) **EP 2 838 872 B1**
(45) Veröffentlichungstag und Bekanntmachung des Hinweises auf die Patenterteilung: **08.06.2016**
(21) Anmeldenummer: 13715241.9
(22) Anmeldetag: 12.04.2013
(51) Int. Cl.: C07C 29/34, C07C 31/125, B01J 31/18

(54) **VERFAHREN ZUR HERSTELLUNG VON VERZWEIGTEN ALKOHOLEN**
METHOD FOR PRODUCING BRANCHED ALCOHOLS
PROCÉDÉ DE FABRICATION D'ALCOOLS RAMIFIÉS

(30) Priorität: 20.04.2012 EP 12164967
(43) Veröffentlichungstag der Anmeldung: 25.02.2015
(73) Patentinhaber: BASF SE, 67056 Ludwigshafen am Rhein (DE)
(72) Erfinder: SCHAUB, Thomas, 67433 Neustadt (DE); DIMITROVA, Pepa, 67550 Worms (DE); PACIELLO, Rocco, 67098 Bad Dürkheim (DE); BAUER, Frederic, 67146 Deidesheim (DE)
(74) Vertreter: BASF IP Association
(86) Internationale Anmeldenummer: PCT/EP2013/057654
(87) Internationale Veröffentlichungsnummer: WO 2013/156399

(56) Entgegenhaltungen:
- EP-A1- 2 221 289
- US-A- 3 479 412
- CARLINI ET AL.: "Selective synthesis of isobutanol by means of the Guerbet reaction", JOURNAL OF MOLECULAR CATALYSIS A: CHEMICAL, Bd. 206, 2003, Seiten 409-418, XP002684139,

## Beschreibung

Die vorliegende Erfindung betrifft ein Verfahren zur Herstellung von verzweigten Alkoholen der allgemeinen Formel (I) 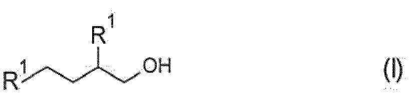 wobei die Gruppen R¹ verschieden sind oder vorzugsweise gleich und gewählt aus C₂-C₃-Alkyl, linear oder verzweigt, unter Verwendung von mindestens einem Alkohol der Formel (II)

R¹-CH₂-CH₂-OH (II)

in homogener Phase
in Gegenwart von mindestens einer Base,
dadurch gekennzeichnet, dass man mindestens eine Ru(II)-haltige Komplexverbindung einsetzt, in der das Ru(II) mindestens einen Liganden L¹ hat, der mindestens zweizähnig ist, wobei mindestens eine Koordinationsstelle von L¹ ein Stickstoffatom ist.

Verzweigte Fettalkohole finden vielseitige Verwendungen als Zwischenprodukte, beispielsweise zur Herstellung von Tensiden. Es ist daher von Interesse, wirtschaftliche Verfahren zur Herstellung von verzweigten Fettalkoholen und insbesondere von in 2-Position verzweigten Fettalkoholen zu entwickeln, Von besonderem Interesse sind dabei Verfahren zur Herstellung von Guerbet-Alkoholen, die neben der Verzweigung in 2-Position weitere Verzweigungen aufweisen.

Aus J. Org. Chem. 2006, 71, 8306 ist bekannt, dass man mit Hilfe von Iridium-Komplexen Guerbet-Reaktionen katalysieren kann. Speziell ist aus der zitierten Stelle bekannt, dass man mit Hilfe von [Cp*lrCl₂]₂ und 1,7-Oktadien und Kalium-tert.-Butanolat in p-Xylol als Lösungsmittel den verzweigten Alkohol Isoamylalkohol zum entsprechenden Guerbetalkohol dimerisieren kann (Cp*: Pentamethylcyclopentadienyl). Die Ausbeute ist jedoch nicht optimal, und Iridiumverbindungen sind teuer.

Aus US 3,479,412 ist bekannt, man mit Hilfe von bestimmten Platin-, Ruthenium- und Rhodium-Katalysatoren Guerbet-Reaktionen katalysieren kann. Beispielhaft wird eine Guerbet-Reaktion offenbart, die durch einen Tributyl-Posphin-Komplex von Ruthenium katalysiert wird.

In US 3,514,493 wird die Herstellung von 2-Ethylhexanol und von 2-Butyloktanol mit Hilfe von geträgerten Metallen, beispielsweise auf Aktivkohle geträgertem Palladium oder Ruthenium, offenbart. In J. Organomet. Chem. 1972, 37, 385 wird vorgeschlagen, dass man mit Hilfe von RuCl₃ mit bestimmten Phosphan-Liganden in homogener Phase Guerbet-Alkohole machen kann.

Der Einsatz der in J. Organomet. Chem. 1972, 37, 385 gezeigten Katalysatoren zur Herstellung von Guerbet-Alkoholen, die neben der Verzweigung in 2-Position weitere oder andere Verzweigungen aufweisen, ist jedoch nicht erfolgreich gewesen. Insbesondere der Versuch, auf Basis von Fuselölen hergestellter sogenannter "biobasierter Isoamylalkohole" einen Guerbet-Alkohol herzustellen, gelingt nicht.

Es bestand also die Aufgabe, ein vielseitiges Verfahren bereit zu stellen, mit dessen Hilfe sich auch solche Guerbet-Alkohole herstellen lassen, die neben der Verzweigung in 2-Position weitere oder andere Verzweigungen aufweisen. Es bestand weiterhin die Aufgabe, Katalysatoren bereit zu stellen, die zur Herstellung von Guerbet-Alkoholen und insbesondere von solchen Guerbet-Alkoholen geeignet sind, die neben der Verzweigung in 2-Position weitere oder andere Verzweigungen aufweisen.

Dementsprechend wurde das eingangs definierte Verfahren gefunden, kurz auch erfindungsgemäßes Verfahren genannt.

Das erfindungsgemäße Verfahren geht aus von mindestens einem Alkohol der allgemeinen Formel (II)

R¹-CH₂-CH₂-OH (II)

in der R¹ gewählt wird aus C₂-C₃-Alkyl, linear oder - soweit möglich - verzweigt, also Ethyl, n-Propyl und Isopropyl, bevorzugt sind Ethyl und Isopropyl, ganz besonders bevorzugt Isopropyl.

Alkohol der allgemeinen Formel (II) kann man in reiner Form oder in Form von Gemischen einsetzen, insbesondere in Form von Isomerengemischen, insbesondere in Form von Gemischen mit mindestens einem isomeren Alkohol. Dabei können im Falle von R¹ = Propyl der oder die isomeren Alkohole der Formel (II) entsprechen. In einer besonderen Variante der vorliegenden Erfindung setzt man Alkohol der allgemeinen Formel (II) im Gemisch mit mindestens einem solchen isomeren Alkohol ein, der nicht der Formel (II) entspricht.

Beispiel für einen geeigneten isomeren Alkohol von Isoamylalkohol (R¹ = Isopropyl) ist 2-Methylbutanol. Dieser reagiert mit Isoamylalkohol vorzugsweise zu einem Alkohol der Formel (la)

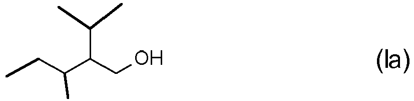

In einer Ausführungsform der vorliegenden Erfindung setzt man Alkohol der Formel (II) in einer Mischung mit 0,1 bis 25 mol-% von mindestens einem isomeren Alkohol ein, der der Formel (II) entsprechen kann, aber vorzugsweise nicht entspricht.

Alkohol der allgemeinen Formel (II) und insbesondere Mischungen von Alkohol der allgemeinen Formel (II) mit einem oder mehreren seiner Isomeren kann man durch Synthese oder aufgrund von biologischen Rohstoffen herstellen, beispielsweise durch Fermentation oder anderen biologischen Abbau von Sacchariden.

Das erfindungsgemäße Verfahren führt man in homogener Phase durch, das heißt, dass man den Katalysator nicht in auf einem festen Träger abgeschiedener Form einsetzt und dass man keine Emulsion erzeugt, in der die Reaktionspartner miteinander reagieren. Wohl aber ist es im Rahmen der vorliegenden Erfindung möglich, dass Alkohol der allgemeinen Formel (II) oder mindestens eines seiner Isomeren zumindest partiell in der Gasphase vorliegen.

Der oder die Katalysatoren sind dabei vollständig oder zumindest überwiegend in der Reaktionsmischung gelöst, beispielsweise zu mindestens 90 mol-%, bevorzugt zu mindestens 95 mol-%, bezogen auf Ru(II).

Man kann das erfindungsgemäße Verfahren in Gegenwart von mindestens einem Lösungsmittel durchführen, das von Alkohol der allgemeinen Formel (II) verschieden ist, beispielsweise in Gegenwart von aromatischen Lösungsmitteln wie beispielsweise para-Xylol, ortho-Xylol, meta-Xylol, Isomerengemischen von Xylol, Mesitylen, oder in Gegenwart von Toluol, Ethylbenzol oder von aliphatischen oder cycloaliphatischen Lösungsmitteln wie beispielsweise n-Hexan, n-Heptan, n-Oktan, n-Nonan, n-Dodecan oder Dekalin. Es ist bevorzugt, das erfindungsgemäße Verfahren ohne Zusatz von Lösungsmittel, das von Alkohol der allgemeinen Formel (II) verschieden ist, durchzuführen.

Man führt das erfindungsgemäße Verfahren in Gegenwart von mindestens einem Katalysator durch, den man vor der eigentlichen Guerbet-Reaktion oder vorzugsweise während der Durchführung des erfindungsgemäßen Verfahrens *in situ* herstellen kann. Als Katalysator setzt man mindestens eine Ru(II)-haltige Komplexverbindung ein, in der das Ru(II) mindestens einen Liganden L¹ hat, der mindestens zweizähnig ist, vorzugsweise zweizähnig oder dreizähnig, wobei mindestens eine Koordinationsstelle von L¹ ein Stickstoffatom ist. Derartige Liganden werden im Rahmen der vorliegenden Erfindung auch kurz "L¹" oder "Ligand L¹" genannt.

In einer Ausführungsform der vorliegenden Erfindung ist Ligand L¹ über zwei, drei oder vier Stickstoffatome mit Ru(II) koordiniert, bevorzugt über zwei oder drei, und L¹ weist keine von Stickstoff verschiedenen Koordinationsstellen auf. Beispiel für einen zweizähnigen Liganden L¹, der über zwei Stickstoffatome mit Ru(II) koordiniert und keine von Stickstoff verschiedenen Koordinationsstellen aufweist, ist 2,2'-Bipyridyl.

In einer anderen Ausführungsform der vorliegenden Erfindung ist Ligand L¹ über zwei oder drei Koordinationsstellen mit Ru(II) koordiniert, von denen eine oder zwei Koordinationsstelle(n) von Stickstoff verschieden ist/sind und die übrige(n) sind/ist Stickstoffatom(e). Von Stickstoff verschiedene Koordinationsstellen von Ligand L¹ sind gewählt aus Phosphor-Atomen, Sauerstoffatomen, Schwefel-Atomen und insbesondere Carben-Kohlenstoff-Atomen.

Stickstoffatome, die an Ru(II) koordinieren, sind dabei vorzugsweise gewählt aus tertiären Amin-Stickstoff-Atomen, die Teil eines Heterocyclus' sind, und Stickstoffatomen, die Teil einer tertiären Aminogruppe sind, die nicht Teil eines Heterocyclus' ist.

In einer Ausführungsform der vorliegenden Erfindung wählt man L¹ aus Verbindungen der allgemeinen Formel (III) 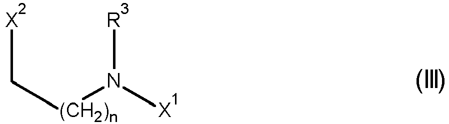 wobei die Variablen wie folgt gewählt sind:
- R³: gewählt aus
Wasserstoff,
C₁-C₁₀-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; bevorzugt n-C₁-C₄-Alkyl wie Methyl, Ethyl, n-Propyl, n-Butyl, insbesondere Methyl;
C₃-C₁₀-Cycloalkyl, beispielsweise Cyclopropyl, Cyclobutyl, Cyclopentyl, Cyclohexyl, Cycloheptyl, Cyclooktyl, Cyclononyl und Cyclodecyl, bevorzugt C₅-C₇-Cycloalkyl, jeweils unsubstituiert oder ein- oder mehrfach substituiert, beispielsweise mit Methyl, Methoxy oder Ethyl,
- n: gewählt aus null und eins,
- X¹: gewählt aus
Wasserstoff,
C₁-C₅-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, insbesondere Methyl oder Isopropyl, und
(CH₂)ₙ₊₁-X²,
- X²: gewählt aus NR⁴R⁵, 2-Pyridyl und Imidazol-2-ylidenyl der Formel

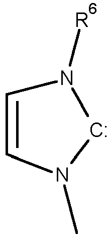

R⁴, R⁵ verschieden oder vorzugsweise gleich und gewählt aus
C₁-C₁₀-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; bevorzugt tert.-Butyl oder n-C₁-C₄-Alkyl wie Methyl, Ethyl, n-Propyl, n-Butyl, insbesondere tert.-Butyl oder Methyl;
C₃-C₁₀-Cycloalkyl, beispielsweise Cyclopropyl, Cyclobutyl, Cyclopentyl, Cyclohexyl, Cycloheptyl, Cyclooktyl, Cyclononyl und Cyclodecyl, bevorzugt C₅-C₇-Cycloalkyl, jeweils unsubstituiert oder ein- oder mehrfach substituiert, beispielsweise mit Methyl, Methoxy oder Ethyl,
Benzyl und
Phenyl, unsubstituiert oder ein- oder mehrfach substituiert mit C₁-C₃-Alkyl, beispielsweise para-Methylphenyl, para-Ethylphenyl, 2,6-Dimethylphenyl, 2,4,6-Trimethylphenyl, 2,6-Diethylphenyl, 2,6-Diisopropylphenyl und 2-Methyl-4-isopropylphenyl,
R⁶ gewählt aus C₁-C₁₀-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; bevorzugt n-C₁-C₄-Alkyl wie Methyl, Ethyl, n-Propyl, n-Butyl, insbesondere Methyl;
C₃-C₁₀-Cycloalkyl, beispielsweise Cyclopropyl, Cyclobutyl, Cyclopentyl, Cyclohexyl, Cycloheptyl, Cyclooktyl, Cyclononyl und Cyclodecyl, bevorzugt C₅-C₇-Cycloalkyl, jeweils unsubstituiert oder ein- oder mehrfach substituiert, beispielsweise mit Methyl, Methoxy oder Ethyl,
Benzyl und
Phenyl, unsubstituiert oder ein- oder mehrfach substituiert mit C₁-C₃-Alkyl, beispielsweise para-Methylphenyl, para-Ethylphenyl, 2,6-Dimethylphenyl, 2,4,6-Trimethylphenyl, 2,6-Diethylphenyl, 2,6-Diisopropylphenyl und 2-Methyl-4-isopropylphenyl.

In einer Ausführungsform der vorliegenden Erfindung wählt man L¹ aus Verbindungen der allgemeinen Formel (IV) 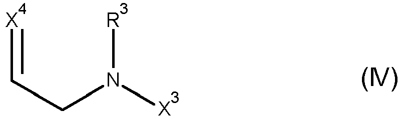 wobei die Variablen wie folgt gewählt sind:
- R³: gewählt aus
Wasserstoff,
C₁-C₁₀-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; bevorzugt n-C₁-C₄-Alkyl wie Methyl, Ethyl, n-Propyl, n-Butyl, insbesondere Methyl
C₃-C₁₀-Cycloalkyl, beispielsweise Cyclopropyl, Cyclobutyl, Cyclopentyl, Cyclohexyl, Cyc-loheptyl, Cyclooktyl, Cyclononyl und Cyclodecyl, bevorzugt C₅-C₇-Cycloalkyl, jeweils un-substituiert oder ein- oder mehrfach substituiert, beispielsweise mit Methyl, Methoxy oder Ethyl,
Benzyl und
Phenyl, unsubstituiert oder ein- oder mehrfach substituiert mit C₁-C₃-Alkyl, beispielsweise para-Methylphenyl, para-Ethylphenyl, 2,6-Dimethylphenyl, 2,4,6-Trimethylphenyl, 2,6-Diethylphenyl, 2,6-Diisopropylphenyl und 2-Methyl-4-isopropylphenyl,
- X³: gewählt aus
Wasserstoff,
C₁-C₅-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl; bevorzugt n-C₁-C₄-Alkyl wie Methyl, Ethyl, n-Propyl, n-Butyl, insbesondere Methyl, und
CH₂=X⁴,
- X⁴: gewählt aus NR⁴, und
- R⁴: gewählt aus
C₁-C₁₀-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, 2-Ethylhexyl, n-Nonyl, n-Decyl; bevorzugt tert.-Butyl oder n-C₁-C₄-Alkyl wie Methyl, Ethyl, n-Propyl, n-Butyl, insbesondere Methyl oder tert.-Butyl,
C₃-C₁₀-Cycloalkyl, beispielsweise Cyclopropyl, Cyclobutyl, Cyclopentyl, Cyclohexyl, Cycloheptyl, Cyclooktyl, Cyclononyl und Cyclodecyl, bevorzugt C₅-C₇-Cycloalkyl, jeweils unsubstituiert oder ein- oder mehrfach substituiert, beispielsweise mit Methyl, Methoxy oder Ethyl,
Benzyl und
Phenyl, unsubstituiert oder ein- oder mehrfach substituiert mit C₁-C₃-Alkyl, beispielsweise para-Methylphenyl, para-Ethylphenyl, 2,6-Dimethylphenyl, 2,4,6-Trimethylphenyl, 2,6-Diethylphenyl, 2,6-Diisopropylphenyl und 2-Methyl-4-isopropylphenyl.

In einer Ausführungsform der vorliegenden Erfindung wählt man L¹ aus Verbindungen der allgemeinen Formel (V) 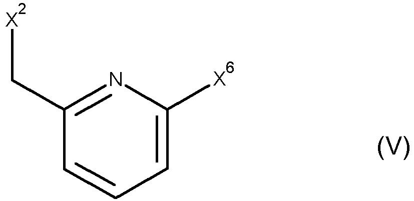 wobei die Variablen wie folgt gewählt sind:
- X⁶: gewählt aus Wasserstoff,
C₁-C₅-Alkyl, beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl; bevorzugt n-C₁-C₄-Alkyl wie Methyl, Ethyl, n-Propyl, n-Butyl, insbesondere Methyl, und
CH₂-X²,
- X²: gewählt aus NR⁴R⁵, 2-Pyridyl und Imidazol-2-ylidenyl der Formel

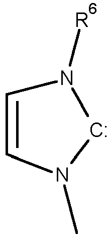

R⁴, R⁵ verschieden oder vorzugsweise gleich und gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl,
R⁶ gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl.
R³, R⁴, R⁵ und R⁶ sind wie vorstehend näher definiert.

In einer Ausführungsform der vorliegenden Erfindung wählt man L¹ aus Verbindungen der allgemeinen Formel (VI) 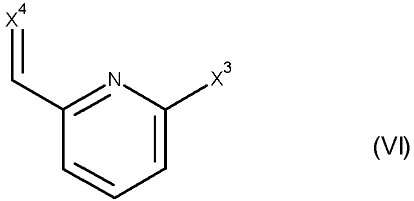 wobei die Variablen wie folgt gewählt sind:
- X³: gewählt aus Wasserstoff, C₁-C₅-Alkyl und CH₂=X⁴,
- X⁴: gleich oder gegebenenfalls verschieden, vorzugsweise gleich, und gewählt aus N-R⁴,
- R⁴: C₁-C₁₀-Alkyl, C₃-C₈-Cycloalkyl, Benzyl oder Phenyl, unsubstituiert oder ein- oder mehrfach substituiert mit C₁-C₃-Alkyl.

In einer Ausführungsform der vorliegenden Erfindung wählt man L¹ aus Verbindungen der allgemeinen Formel (VII) 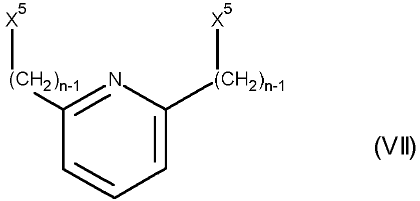 wobei die Variablen wie folgt gewählt sind:
n verschieden oder vorzugsweise gleich und jeweils null oder eins
X⁵ jeweils gleich und gewählt aus NR⁴R⁵, 2-Pyridyl und Imidazol-2-ylidenyl der Formel 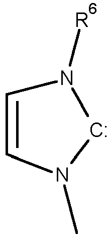
R⁴, R⁵ verschieden oder gleich und gewählt aus Wasserstoff, C₁-C₁₀-Alkyl, C₃-C₁₀-Cycloalkyl, Benzyl und Phenyl, unsubstituiert oder ein- oder mehrfach substituiert mit C₁-C₃-Alkyl,
R⁶ gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl.
R³, R⁴, R⁵ und R⁶ sind wie vorstehend näher definiert.

Besonders bevorzugte Beispiele für Liganden L¹ sind solche der Formel (VII.1) 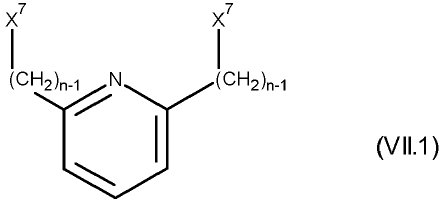 bei denen die Gruppen (CH₂)ₙ₋₁ -X⁷ jeweils gleich sind und gewählt aus 2-Pyridyl (also jeweils n = null),
CH₂-N(CH₃)₂, CH₂-N(C₂H₅)₂, CH₂-N(n-C₃H₇)₂, CH₂-N(n-C₄H₉)₂, CH₂-N(iso-C₃H₇)₂, CH₂-N(tert.-C₄H₉)₂, CH₂-N(n-C₅H₁₁)₂, CH₂-N(n-C₆H₁₃)₂, CH₂-N(n-C₈H₁₇)₂, CH₂-N(C₆H₅)₂, CH₂-N(CH₂-C₆H₅)₂, und CH₂-N(cyclo-C₆H₁₁)₂, (also jeweils n = 1), und 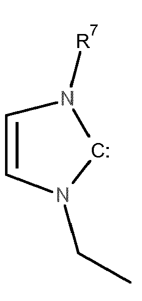 (also jeweils n = 1), mit R⁷ gewählt aus
Methyl, Ethyl, n-Propyl, iso-Propyl, n-Butyl, n-Pentyl, n-Hexyl, n-Heptyl, n-Octyl, bevorzugt Iso-propyl oder n-C₁-C₄-Alkyl wie Methyl, Ethyl, n-Propyl, n-Butyl, insbesondere Methyl,
Cyclohexyl und
Phenyl, unsubstituiert oder ein- oder bis zu dreifach substituiert mit gleichem oder verschiedenem C₁-C₃-Alkyl, beispielsweise para-Methylphenyl, 2,6-Dimethylphenyl, 2,4,6-Trimethylphenyl, 2,6-Diethylphenyl, 2,6-Diisopropylphenyl und 2-Methyl-4-isopropylphenyl.

Andere besonders bevorzugte Beispiele für Liganden L¹ sind solche der Formel (VI.1) 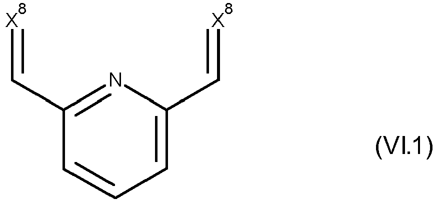 in denen X⁸ jeweils gleich sind und gewählt aus N-CH₃, N-C₂H₅, N-n-C₃H₇, N-n-C₄H₉, N-iso-C₃H₇, N-n-C₅H₁₁, N-n-C₆H₁₃, N-n-C₈H₁₇, N-CH₂-C₆H₅ und N-cyclo-C₆H₁₁, und N-Phenyl, unsubstituiert oder ein- oder bis zu dreifach substituiert mit gleichem oder verschiedenem C₁-C₃-Alkyl, beispielsweise N-para-Methylphenyl, N-2,6-Dimethylphenyl, N-2,4,6-Trimethylphenyl, N-2,6-Diethylphenyl, N-2,6-Diisopropylphenyl und N(2-methyl-4-isopropylphenyl).

Ein ganz besonders bevorzugter Ligand L¹ ist 2,6-bis-2-Pyridylpyridin, im Rahmen der vorliegenden Erfindung auch kurz "Terpyridyl" genannt.

In einer Ausführungsform der vorliegenden Erfindung kann Ru(II)-haltige Komplexverbindung mindestens einen weiteren Liganden aufweisen, gewählt aus CO, Pseudohalogeniden, organischen Carbonylverbindungen, Aromaten, Olefinen, Phosphanen, Hydrid und Halogeniden.

Unter "mindestens einem weiteren Liganden" ist dabei ein Ligand zu verstehen, der von Ligand L¹ verschieden ist. Beispiele für weitere Liganden sind
- CO (Kohlenmonoxid),
- Pseudohalogenid, insbesondere Cyanid, Isocyanat und Rhodanid,
- organische Carbonylverbindungen, beispielsweise Ketone, bevorzugt organische Dicarbonylverbindungen wie Acetylacetonat, 1-Phenylbutan-1,3-dion, Acetessigester
- Aromaten, die elektrisch geladen sein können oder ungeladen. Bevorzugte Beispiele für ungeladene Aromaten sind Benzol, Toluol, para-Xylol, Hexamethylbenzol und paracymen. Bevorzugte Beispiele für elektrisch geladene Aromaten sind negativ geladene Aromaten, insbesondere Cyclopentadienyl, Indenyl, 4,5-Benzindenyl und Cp* (Pentamethylcyclopentadienyl),
- Olefine, elektrisch neutral oder als Anion, beispielsweise COD (1,5-Cyclooktadienyl), Allyl oder Methallyl (2-Methylallyl),
- Phosphanen, beispielsweise Mono-, Di- oder Triphosphane, vorzugsweise Monophosphanen, insbesondere tertiären aromatischen Phosphanen, beispielsweise Triphenylphosphan,
- Hydrid und
- Halogenen, beispielsweise Bromid und insbesondere Chlorid.

Bespiele für als weiterer Ligand geeignete Phosphane sind solche, die mindestens einen unverzweigten oder verzweigten C₁-C₁₂-Alkylrest, mindestens einen C₃-C₁₂-Ccycloalkylrest oder mindestens einen aromatischen Rest mit bis zu 24 C-Atomen aufweisen. Beispiele für C₁-C₁₂-Alkylreste sind Methyl, Ethyl, 1-Propyl, 2-Propyl, 1-Butyl, 2-Butyl, 1-(2-Methyl)propyl, 2-(2-Methyl)propyl, 1-Pentyl, 1-Hexyl, 1-Heptyl, 1-Octyl, 1-Nonyl, 1-Decyl, 1-Undecyl, 1-Dodecyl, 1-(2-Methyl)-pentyl, 1-(2-Ethyl)-hexyl, 1-(2-n-Propyl)heptyl. Bevorzugte C₁-C₁₂-Alkylreste sind gewählt aus Ethyl, 1-Butyl, sec.-Butyl und 1-Octyl.

Beispiele für C₃-C₁₂-Cycloalkylreste sind insbesondere gewählt aus C₄-C₈-Cyloalkylreste, verzweigt oder unverzweigt, wie Cyclopentyl, Cyclohexyl, Cycloheptyl, Cyclooctyl, Methylcyclopen-tyl, beispielsweise 2-Methylcyclopentyl, 3-Methylcyclopentyl, weiterhin 2,5-Dimethylcyclopentyl (syn, anti oder als Isomerengemisch), 2-Methylcyclohexyl, 3-Methylcyclohexyl, 4-Methylcyclohexyl, 2,6-Dimethylcyclohexyl (syn, anti oder als Isomerengemisch), Norbonyl und - CH₂-C₆H₁₁. Bevorzugter C₃-C₁₂-Cycloalkylrest ist Cyclohexyl.

In einer bevorzugten Variante wählt man als weiteren Ligand ein Phosphan, das zwei, besonders bevorzugt drei gleiche Reste trägt, beispielsweise Tri-n-butylphosphan, Tri-sec.-butylphosphan, Tricyclohexylphosphan oder Tri-n-octylphosphan.

In einer Ausführungsform wählt man als Substituenten von als weiterem Ligand geeignetem Phosphan mindestens einen aromatischen Rest, beispielsweise 9-Anthracenyl, bevorzugt drei gleiche aromatische Reste, beispielsweise Phenyl, 2-Tolyl, 3-Tolyl, para-Tolyl, Xylyl, 1-Naphthyl, 2-Naphthyl, 1-Binaphthyl, para-Anisyl, 2-Ethylphenyl, 3-Ethylphenyl, para-Ethylphenyl, 2-Chlorphenyl, para-Chlorphenyl, 2,6-Dichlorphenyl, oder mindestens einen heteroaromatischen Rest. Beispiele für heteroaromatische Reste sind Thienyl, Benzothienyl, 1-Naphthothienyl, Thianthrenyl, Furyl, Benzofuryl, Pyrrolyl, Imidazolyl, Pyrazolyl, Pyridyl, Pyrazinyl, Pyrimidinyl, Pyridazinyl, Indolyl, Isoindolyl, Indazolyl, Purinyl, Isoquinolinyl, Quinolinyl, Acridinyl, Naphthyridinyl, Quinoxalinyl, Quinazolinyl, Cinnolinyl, Piperidinyl, Carbolinyl, Thiazolyl, Oxazolyl, Isothiazolyl, Isoxazolyl. Die Heteroarylgruppen können unsubstituiert oder substituiert sein, mit einem oder mehreren Substituenten, die vorstehend unter C₁-C₁₂-Alkyl definiert sind.

In einer anderen bevorzugten Variante wählt man als weiteren Ligand ein mehrzähniges Phosphan, beispielsweise eines mit der Gruppierung >P-CH₂-CH₂-P(C₁-C₁₀-Alkyl)-CH₂CH₂-P<, besonders bevorzugt mit der Gruppierung >P-CH₂-CH₂-P<. Beispiel ist 1,2-Bis(dicyclohexylphosphino)ethan.

In einer Ausführungsform der vorliegenden Erfindung setzt man 0,001 bis 5 mol-% Ru(II) ein, bezogen auf Alkohol der allgemeinen Formel (II).

Das erfindungsgemäße Verfahren führt man in Gegenwart von mindestens einer Base durch. Als Basen sind Brönsted-Basen bevorzugt. Als Beispiele für geeignete Basen seien genannt: LiOH, NaOH, KOH, LiH, NaH, KH, Ca(OH)₂, CaH₂, LiAIH₄, NaBH₄, LiBH₄, Na₂CO₃, NaHCO₃, Li₂CO₃, LiHCO₃, K₂CO₃, KHCO₃, K₃PO₄, Na₃PO₄, n-Buthyllithium, tert.-BuLi, Methyllithium, Phenyllithium, Lithiummethanolat, Lithiumethanolat, LiO-n-C₃H₇, LiO-iso-C₃H₇, LiO-n-C₄H₉, LiO-iso-C₄H₉, LiO-n-C₅H₁₁, LiO-iso-C₅H₁₁, LiO-n-C₆H₁₃, LiO-iso-C₆H₁₃, Lithium-n-heptanolat, Lithium-n-oktanolat, Lithium-benzylat, Lithiumphenolat, Kaliummethanolat, Kaliumethanolat, KO-n-C₃H₇, KO-iso-C₃H₇, KO-n-C₄H₉, KO-iso-C₄H₉, KO-tert.-C₄H₉, KO-n-C₅H₁₁, KO-iso-C₅H₁₁, KO-n-C₆H₁₃, KO-iso-C₆H₁₃, Kalium-n-heptanolat, Kalium-n-oktanolat, Kalium-benzylat, Kalium-phenolat, Natriummethanolat, Natriumethanolat, NaO-n-C₃H₇, NaO-iso-C₃H₇, NaO-n-C₄H₉, NaO-iso-C₄H₉, NaO-tert.-C₄H₉, NaO-n-C₅H₁₁, NaO-iso-C₅H₁₁, NaO-n-C₆H₁₃,
NaO-iso-C₆H₁₃, Natrium-n-heptanolat, Natrium-n-oktanolat, Natrium-benzylat, Natriumphenolat, KN(SiMe₃)₂, LiN(SiMe₃)₂, NaN(SiMe₃)₂, NH₃ und Amine der Formel (R⁸)ₐNH₃₋ₐ, wobei a gewählt wird aus 1, 2 und 3, und R⁸ = gleich oder verschieden und unabhängig voneinander unsubstituiertes oder zumindest monosubstituiertes C₁-C₁₀-Alkyl, (-C₁-C₄-Alkyl-P(Phenyl)₂), C₃-C₁₀-Cycloalkyl, C₃-C₁₀-Heterocyclyl, wobei unter C₃-C₁₀-Heterocyclyl solche cyclische Gruppen zu verstehen sind, die 3 bis 10 C-Atome und mindestens ein Heteroatom aufweisen, ausgewählt aus S, weiterhin C₅-C₁₄-Aryl oder C₅-C₁₀-Heteroaryl, wobei C₅-C₁₀-Heteroaryl mindestens ein Heteroatom aufweist, ausgewählt aus N, O und S.

In einer Ausführungsform der vorliegenden Erfindung setzt man insgesamt 0,01 bis 50 Gew.-% Base ein, bevorzugt 0,5 bis 15 Gew.-%, bezogen auf gesamten eingesetzten Alkohol der Formel (II).

In einer Ausführungsform der vorliegenden Erfindung ist die Reaktionsmischung bei Reaktionstemperatur flüssig.

In einer Ausführungsform der vorliegenden Erfindung führt man das erfindungsgemäße Verfahren bei einer Temperatur im Bereich von 80 bis 200°C, bevorzugt 100 bis 200°C, besonders bevorzugt im Bereich von 110 bis 170°C durch.

In einer Ausführungsform der vorliegenden Erfindung führt man das erfindungsgemäße Verfahren in Gegenwart von mindestens einem inerten Gas durch. Geeignete inerte Gase sind gewählt aus Stickstoff und Edelgasen, insbesondere Argon. In einer anderen Ausführungsform der vorliegenden Erfindung führt man das erfindungsgemäße Verfahren in Gegenwart von Wasserstoff durch. In einer weiteren Ausführungsform führt man das erfindungsgemäße Verfahren in Gegenwart einer Mischung von Wasserstoff und mindestens einem inerten Gas durch.

In einer Ausführungsform der vorliegenden Erfindung führt man das erfindungsgemäße Verfahren bei einem Druck im Bereich von 0,1 bis 5 MPa absolut, welcher der Eigendruck des Lösungsmittels bzw. des Alkohols der allgemeinen Formel (II) bei der Reaktionstemperatur bzw. der Druck eines Gases wie Stickstoff, Argon oder Wasserstoff sein kann, durch. Bevorzugt wird das erfindungsgemäße Verfahren bei einem Gesamtdruck bis 3 MPa absolut, insbesondere bevorzugt bei einem Gesamtdruck von 0,1 bis 1 MPa absolut durchgeführt.

Zur Durchführung des erfindungsgemäßen Verfahrens kann man beispielsweise so vorgehen, dass man Alkohol der allgemeinen Formel (II) mit Base und mindestens einer Ru(II)-haltigen Komplexverbindung vermischt, die mindestens einen Liganden L¹ aufweist.

In einer anderen Ausführungsform der vorliegenden Erfindung erzeugt man den Katalysator in situ. Darunter soll zu verstehen sein, dass man Ru(II)-haltige Komplexverbindung, die mindestens einen Liganden L¹ aufweist, nicht isoliert, sondern ohne weitere Aufarbeitung durch Vermischen einer Ru(II)- oder Ru(III)-Ausgangsverbindung und Ligand L¹ erzeugt, beispielsweise indem man Ru(II)- oder Ru(III)-Ausgangsverbindung und Ligand L¹ mit Base und Alkohol der allgemeinen Formel (II) vermischt, gegebenenfalls in Gegenwart von einem Reduktionsmittel. Geeignete Ru(II)- bzw. Ru(III)-Ausgangsverbindungen sind beispielsweise Ru(p-Cymol)Cl₂]₂, [Ru(benzol)Cl₂]_{y}, [Ru(CO)₂Cl₂]_{y}, wobei y jeweils im Bereich von 1 bis 1.000 liegt, [Ru(CO)₃Cl₂]₂, [Ru(COD)(allyl)], RuCl₃·H₂O, [Ru(acetylacetonat)₃], [Ru(DMSO)₄Cl₂], [Ru(cyclopentadienyl)(CO)₂Cl], [Ru(cyclopentdienyl)(CO)₂H], [Ru(cyclopentadienyl)(CO)₂]₂, [Ru(Cp)(CO)₂Cl], [Ru(Cp*)(CO)₂H], [Ru(Cp*)(CO)₂]₂, [Ru(indenyl)(CO)₂Cl], [Ru(indenyl)(CO)₂H], [Ru(indenyl)(CO)₂]₂, Ruthenocen, [Ru(COD)Cl₂]₂, [Ru(Cp*)(COD)Cl], [Ru₃(CO)₁₂], [Ru(PPh₃)₄(H)₂], [Ru(PPh₃)₃(Cl)₂], [Ru(PPh₃)₃(CO)(Cl)₂], [Ru(PPh₃)₃(CO)(Cl)(H)], [Ru(PPh₃)₃(CO)(H)₂] und [Ru(cyclooctadienyl)(methylallyl)₂].

Dabei bedeuten Cp*: Pentamethylcyclopentadienyl, COD: 1,5-Cyclooktadienyl, methylallyl: 2-Methylallyl.

Durch die Wahl der Ru(II)- bzw. Ru(III)-Ausgangsverbindung kann man die Auswahl des oder der weiteren Liganden beeinflussen.

In einer Ausführungsform der vorliegenden Erfindung kann man Ligand L¹ und Ru(II)- bzw. Ru(III)-Ausgangsverbindung in stöchiometrischen Anteilen, jeweils bezogen auf Ru(II) bzw. Ru(III), einsetzen. In einer anderen Variante kann man einen Überschuss an Ligand L¹ einsetzen, bezogen auf Ru(II)- bzw. Ru(III) in Ru(II)- bzw. Ru(III)-Ausgangsverbindung, beispielsweise 1,1 bis 5 Moläquivalente L¹ pro Ru(II) bzw. Ru(III).

Bei der Durchführung des erfindungsgemäßen Verfahrens wird *in situ* als Nebenprodukt Wasser gebildet. Es ist bevorzugt, das entstehende Wasser, kurz auch Reaktionswasser genannt, abzutrennen.

In einer Ausführungsform der vorliegenden Erfindung trennt man das Reaktionswasser ab, indem man es mit einem azeotropen Schleppmittel abtrennt, beispielsweise einem der vorstehend genannten Lösungsmittel, insbesondere einem der vorstehend genannten aromatischen Lösungsmittel. In einer bevorzugten Variante geht man so vor, dass man Alkohol der allgemeinen Formel (II) als azeotropes Schleppmittel einsetzt, da er mit Wasser eine Mischungslücke aufweist, um Reaktionswasser abzutrennen bzw. auszukreisen.

Bevorzugt wird während der Reaktion das Reaktionswasser mit Hilfe eines Wasserabscheiders ausgekreist.

Das erfindungsgemäße Verfahren kann man in verschiedensten Reaktionsgefäßen durchführen, in denen Flüssigkeitsreaktionen, gegebenenfalls mit einem Gasraum, durchgeführt werden können. Geeignete Reaktionsgefäße sind beispielsweise in K.D. Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien genannt: Rührkesselreaktoren, Rohrreaktoren und Blasensäulenreaktoren.

Das erfindungsgemäße Verfahren kann man diskontinuierlich, also in Batchfahrweise, oder kontinuierlich oder semikontinuierlich mit Rückführung oder ohne Rückführung durchgeführt werden. Die mittlere Verweilzeit der entstehenden Reaktionsmasse im Reaktionsgefäß kann beispielsweise im Bereich von 15 Minuten bis 100 Stunden betragen.

Ohne dass einer bestimmten Theorie der Vorzug gegeben werden soll, so ist es plausibel, dass das erfindungsgemäße Verfahren im Wesentlichen drei Reaktionen umfasst. Zunächst wird Alkohol der Formel (II) oxidativ dehydriert, und zwar zum Aldehyd. Dann erfolgt eine Aldolkondensation, gefolgt von einer Reduktion.

Durch die Ausführung des erfindungsgemäßen Verfahrens erhält man verzweigten Alkohol der allgemeinen Formel (I) 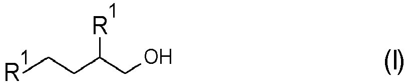 wobei die Gruppen R¹ verschieden sind oder gleich und wie vorstehend definiert.

Wenn man eine Mischung von Alkohol der allgemeinen Formel (II) mit einem oder mehreren Isomeren als Ausgangsmaterial einsetzt, so erhält man üblicherweise ein Gemisch von verzweigten Alkoholen der allgemeinen Formel (I).

In einer Ausführungsform der vorliegenden Erfindung erhält man verzweigten Alkohol der allgemeinen Formel (1.1) 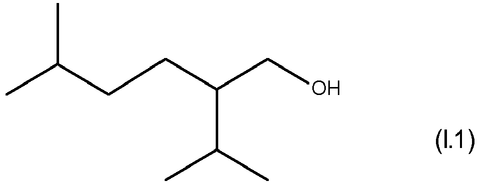 im Gemisch mit Alkohol der Formel (la)

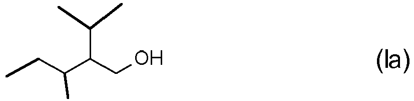

In einer anderen Ausführungsform erhält man verzweigten Alkohol der Formel (I.2) 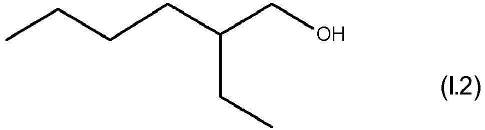 im Gemisch mit Alkohol der Formel (Ia.2)

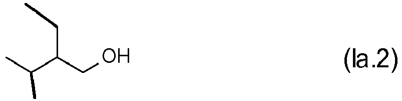

In einer Ausführungsform der vorliegenden Erfindung erhält man als weiteres Nebenprodukt Ester, beispielsweise - wenn man von Isoamylalkohol als Alkohol der Formel (I) ausgeht - einen Ester der Formel

(CH₃)₂CH-(CH₂)-COO-(CH₂)₂-CH(CH₃)₂

In einer Ausführungsform der vorliegenden Erfindung führt man das erfindungsgemäße Verfahren bis zu vollständigem Umsatz von Alkohol der allgemeinen Formel (II) durch. In einer anderen Ausführungsform führt man die Umsetzung nur zu unvollständigem Umsatz durch, beispielsweise zu 8 bis 50 mol-%, bevorzugt bis 30 mol-%, und arbeitet danach auf.

Dabei ist es möglich, Ru(II) zurückzugewinnen und erneut einzusetzen.

Zum Zweck der Aufarbeitung kann man beispielsweise so vorgehen, dass man verzweigten Alkohol der allgemeinen Formel (I) von nicht umgesetztem Alkohol der allgemeinen Formel (II) sowie von Base und Komplexverbindung von Ru(II) durch Destillation abtrennt. Komplexverbindung von Ru(II) und Base verbleibt mit eventuell angefallenen Hochsiedern, beispielsweise Trimerisierungsprodukt von Alkohol der allgemeinen Formel (II), im Sumpf der Destillation zurück und kann wiederverwendet werden. Nicht umgesetzten Alkohol der allgemeinen Formel (II) kann man ebenfalls wieder in die Reaktion zurückführen. Die thermische Abtrennung von Alkohol der allgemeinen Formel (I) sowie von gegebenenfalls gebildetem Ester kann beispielsweise nach an sich bekannten Verfahren erfolgen, bevorzugt in einem Verdampfer oder in einer Destillationseinheit, umfassend Verdampfer und Kolonne(n), die üblicherweise mehrere Böden oder eine Packung oder Füllkörper aufweist bzw. aufweisen.

Durch das erfindungsgemäße Verfahren lassen sich verzweigte Alkohole der Formel (I) in guter Ausbeute und sehr gute Reinheit herstellen. Man kann zu ihrer Herstellung nicht nur von reinem Alkohol der allgemeinen Formel (II) ausgehen, sondern auch Isomerengemische verwenden, beispielsweise solche, die man durch Fermentation oder anderen biologischen Abbau von Sacchariden erhalten kann, insbesondere sogenannte Fuselöle.

Ein weiterer Gegenstand der vorliegenden Erfindung ist die Verwendung von Katalysatoren, enthaltend mindestens eine Ru(II)-haltige Komplexverbindung, in der das Ru(II) mindestens einen Liganden L¹ hat, der mindestens zweizähnig ist, wobei mindestens eine Koordinationsstelle von L¹ ein Stickstoffatom ist, zur Herstellung von Alkoholen der allgemeinen Formel (I) 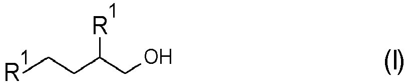 wobei die Gruppen R¹ verschieden sind oder gleich und gewählt aus C₂-C₃-Alkyl, linear oder verzweigt, unter Verwendung von mindestens einem Alkohol der Formel (II)

R¹-CH₂-CH₂-OH (II).

Dabei sind die Variablen wie vorstehend näher definiert.

Dabei ist die erfindungsgemäße Verwendung insbesondere dann bevorzugt, wenn man, aus gehend von Alkohol der Formel (II), der auf Basis von Fuselölen hergestellt ist, also auf Basis sogenannter "biobasierter Isoamylalkohole", verzweigten Alkohol der Formel (I) herzustellen wünscht.

In einer Variante der erfindungsgemäßen Verwendung weist die Ru(II)-haltige Komplexverbindung mindestens einen weiteren Liganden auf, gewählt aus CO, Pseudohalogeniden, organischen Carbonylverbindungen, Aromaten, Olefinen, Phosphanen, Hydrid und Halogeniden. In einer bevorzugten Variante wählt man L¹ aus zweizähnigen und dreizähnigen Liganden, die über Stickstoffatome und gegebenenfalls über ein oder mehr Carben-Kohlenstoffatome mit Ru(II) koordinieren. Beispiele für besonders bevorzugte Liganden L¹ sind vorstehend genannt.

Die vorliegende Erfindung wird durch Arbeitsbeispiele weiter erläutert.

### Arbeitsbeispiele:

### Allgemeine Vorbemerkungen:

Die Beispiele 1.1 bis 1.11 wurden unter Inertbedingungen (Argonüberdeckung) in einem 50 ml-Schlenkkolben mit Rückflusskühler durchgeführt. Man wog Ruthenium(II)-Ausgangsverbindung (0,05 mol-% bzgl. Isoamylalkohol), Ligand L¹, die Base (500 mg KOH; 9,7 mol%) sowie Isoamylalkohol (10 ml, (II.1)) in einer Glovebox in den Schlenkkolben ein. Man erhielt ein Reaktionsgemisch. Man rührte das Reaktionsgemisch bei 130°C für 16 Stunden. Ausbeute und Selektivität an verzweigtem C₁₀-Alkohol der Formel (I.1) wurde durch Gaschromatographie (FI%) bestimmt.

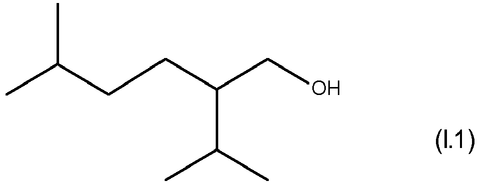

**Tabelle 1: Ergebnisse der Beispiele I.1 bis 1.11**

| Beispiel | Ru(II)-Ausgangsverbindung | L¹ | Umsatz (11.1) | Selektivität (1.1) |
|---|---|---|---|---|
| I.1 | [Ru(PPh₃)(H)₂(CO)] | (VII.1.2.a) | 31,8 % | 92,5 % |
| I.2 | [Ru(PPh₃)(H)₂(CO)] | (VI.1.a) | 29,5 % | 91,9 % |
| I.3 | [Ru(PPh₃)(H)₂(CO)] | (VII.1.1) | 8 % | 75 % |
| I.4 | [Ru(PPh₃)(H)₂(CO)] | (VII. 1.2.a) | 15,9 % | 88,1 % |
| I.5 | [Ru(PPh₃)(H)₂(CO)] | (VI.1.b) | 33,0 % | 85,1 % |
| I.6 | [Ru(PPh₃)(H)₂(CO)] | (VII.1.3.a) | 15,0 % | 74,0 % |
| I.7 | [Ru(PPh₃)(H)₂(CO)] | (VII.1.3.b) | 10,2 % | 100 % |
| I.8 | [Ru(PPh₃)(H)₂(CO)] | (VII.1.3.c) | 23,0 % | 39,6 % |
| I.9 | [Ru(PPh₃)₃(H)(Cl)(CO)] | (VI.1.a) | 31,8 % | 85,5 % |
| I.10 | [Ru(PPh₃)₃(H)(Cl)(Toluol)] | (VI.1.a) | 29,4 % | 75,9 % |
| I.11 | [Ru(PPh₃)₃(Cl)₂(CO)] | (VI.1.a) | 26,3 % | 76,4 % |

| | | | | |
|---|---|---|---|---|
| Folgende Liganden L¹ wurden eingesetzt: (VI.1.a) bzw. (VI.1.b): 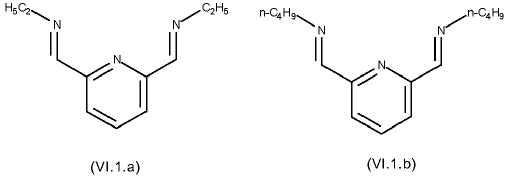 (VII.1.1): 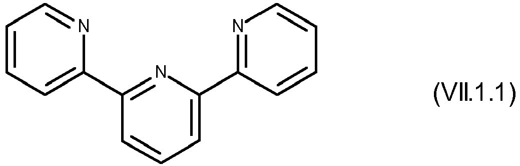 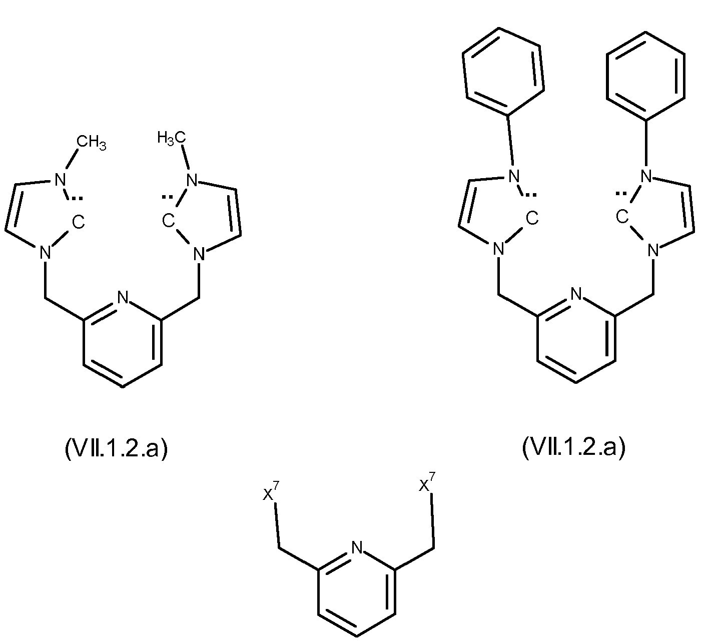 (VII.1.3.a): X⁷ jeweils gleich und N(C₆H₅)₂ (VII. 1.3.b): X⁷ jeweils gleich und N(CH₂-C₆H₅)₂, (VII.1.3.c): X⁷ jeweils gleich und N(tert.-C₄H₉)₂ | | | | |

### Beispiel II:

Unter Inertbedingungen (Glovebox) wurden in einen 250 ml-Dreihalskolben 102 g (1,16 mol) Isoamylalkohol, 5,0 g (89 mmol) KOH, 130 mg (0,46 mmol) [Ru(COD)(Cl)₂]₂ und 250 mg (1,35 mmol) PPh₃ eingewogen. Man erhielt ein Gemisch, das man mit Argon überdeckte. Der 250 ml-Dreihalskolben wurde dann mit einem Rückflusskühler ausgestattet, das Gemisch auf 100°C erwärmt und bei 100°C für zwei Stunden gerührt. Danach wurden 120 mg (0,48 mmol) des Liganden (VI.1.a), gelöst in 2 ml Isoamylalkohol, zugegeben. Das Reaktionsgemisch färbte sich braun. Das braune Reaktionsgemisch wurde dann über einen Zeitraum von 16 Stunden bei einer Ölbadtemperatur von 170°C unter Verwendung eines Wasserabscheiders (Wasserauskreisers) am Rückfluss gekocht. Danach ließ man auch Zimmertemperatur abkühlen. Das Gaschromatogramm des Reaktionsgemisches zeigte einen Umsatz an Isoamylalkohol von 80,8 % und eine Selektivität bezüglich des Alkohols der Formel (1.1) von 87,2 %.

## Patentansprüche

1. Verfahren zur Herstellung von verzweigten Alkoholen der allgemeinen Formel (I) 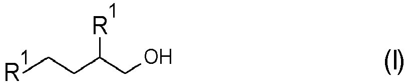 wobei die Gruppen R¹ verschieden sind oder gleich und gewählt aus C₂-C₃-Alkyl, linear oder verzweigt, unter Verwendung von mindestens einem Alkohol der Formel (II)
R¹-CH₂-CH₂-OH (II)
in homogener Phase
in Gegenwart von mindestens einer Base,
**dadurch gekennzeichnet, dass** man mindestens eine Ru(II)-haltige Komplexverbindung einsetzt, in der das Ru(II) mindestens einen Liganden L¹ hat, der mindestens zweizähnig ist, wobei mindestens eine Koordinationsstelle von L¹ ein Stickstoffatom ist.

2. Verfahren nach Anspruch 1, **dadurch gekennzeichnet, dass** man es ohne Zusatz von Lösungsmittel, das von Alkohol der Formel (II) verschieden ist, durchführt.

3. Verfahren nach Anspruch 1 oder 2, **dadurch gekennzeichnet, dass** man es bei Temperaturen im Bereich von 100 bis 200°C durchführt.

4. Verfahren nach einem der Ansprüche 1 bis 3, **dadurch gekennzeichnet, dass** man R¹ wählt aus Ethyl und Isopropyl.

5. Verfahren nach einem der Ansprüche 1 bis 4, **dadurch gekennzeichnet, dass** man L¹ wählt aus zweizähnigen und dreizähnigen Liganden, die über ein oder mehrere Stickstoffatome und gegebenenfalls ein oder mehr Carben-Kohlenstoffatome mit Ru(II) koordinieren.

6. Verfahren nach einem der Ansprüche 1 bis 5, **dadurch gekennzeichnet, dass** Ru(II)-haltige Komplexverbindung mindestens einen weiteren Liganden aufweist, gewählt aus CO, Pseudohalogeniden, organischen Carbonylverbindungen, Aromaten, Olefinen, Phosphanen, Hydrid und Halogeniden.

7. Verfahren nach einem der Ansprüche 1 bis 6, **dadurch gekennzeichnet, dass** man L¹ wählt aus Verbindungen der allgemeinen Formel (III) 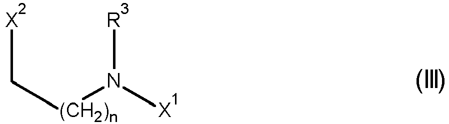 wobei die Variablen wie folgt gewählt sind:
R³ gewählt aus Wasserstoff und C₁-C₅-Alkyl,
n gewählt aus null und eins,
X¹ gewählt aus Wasserstoff, C₁-C₅-Alkyl und (CH₂)ₙ₊₁-X₂,
X² gewählt aus NR⁴R⁵, 2-Pyridyl und Imidazol-2-ylidenyl der Formel 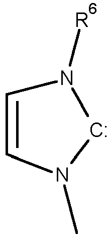
R⁴, R⁵ verschieden oder gleich und gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl,
R⁶ gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl.

8. Verfahren nach einem der Ansprüche 1 bis 6, **dadurch gekennzeichnet, dass** man L¹ wählt aus Verbindungen der allgemeinen Formel (IV) 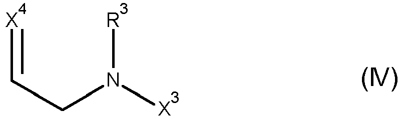 wobei die Variablen wie folgt gewählt sind:
R³ gewählt aus Wasserstoff, C₁-C₁₀-Alkyl, C₃-C₁₀-Cycloalkyl, Benzyl und Phenyl,
X³ gewählt aus Wasserstoff, C₁-C₅-Alkyl und CH₂=X⁴,
X⁴ gewählt aus NR⁴, und
R⁴ gewählt aus C₁-C₁₀-Alkyl und C₃-C₁₀-Cycloalkyl, Benzyl und Phenyl, unsubstituiert oder ein- oder mehrfach substituiert mit C₁-C₃-Alkyl.

9. Verfahren nach einem der Ansprüche 1 bis 6, **dadurch gekennzeichnet, dass** man L¹ wählt aus Verbindungen der allgemeinen Formel (V) 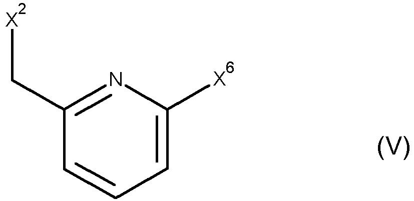 wobei die Variablen wie folgt gewählt sind:
X⁶ gewählt aus Wasserstoff, C₁-C₅-Alkyl und CH₂-X²,
X² gewählt aus NR⁴R⁵, 2-Pyridyl und Imidazol-2-ylidenyl der Formel 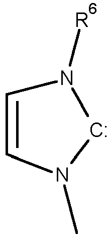
R⁴, R⁵ verschieden oder gleich und gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl,
R⁶ gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl.

10. Verfahren nach einem der Ansprüche 1 bis 6, **dadurch gekennzeichnet, dass** man L¹ wählt aus Verbindungen der allgemeinen Formel (VI) 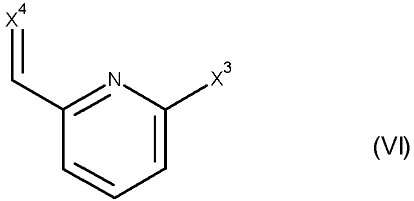 wobei die Variablen wie folgt gewählt sind:
X³ gewählt aus Wasserstoff, C₁-C₅-Alkyl und CH₂=X⁴,
X⁴ verschieden oder gleich und gewählt aus N-R⁴,
R⁴ gewählt aus C₁-C₁₀-Alkyl, C₃-C₈-Cycloalkyl, Benzyl und Phenyl, unsubstituiert oder ein- oder mehrfach substituiert mit C₁-C₃-Alkyl.

11. Verfahren nach einem der Ansprüche 1 bis 10, **dadurch gekennzeichnet, dass** man L¹ wählt aus Verbindungen der Formel (VII) 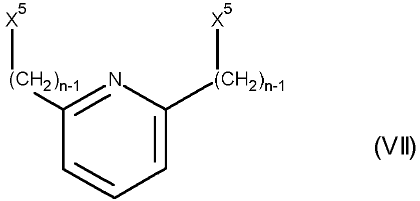 wobei die Variablen wie folgt gewählt sind:
n gleich oder verschieden und jeweils null oder eins
X⁵ jeweils gleich und gewählt aus NR⁴R⁵, 2-Pyridyl und Imidazol-2-ylidenyl der Formel 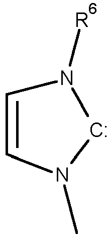
R⁴, R⁵ verschieden oder gleich und gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl,
R⁶ gewählt aus C₁-C₁₀-Alkyl, C₃-C₁₀-Cycloalkyl, Benzyl und Phenyl, unsubstituiert oder ein- oder mehrfach substituiert mit C₁-C₃-Alkyl.

12. Verfahren nach einem der Ansprüche 1 bis 11, **dadurch gekennzeichnet, dass** man den Katalysator in situ erzeugt.

13. Verfahren nach einem der Ansprüche 1 bis 12, **dadurch gekennzeichnet, dass** man Alkohol der Formel (II) als azeotropes Schleppmittel einsetzt.

14. Verwendung von Katalysatoren, enthaltend mindestens eine Ru(II)-haltige Komplexverbindung, in der das Ru(II) mindestens einen Liganden L¹ hat, der mindestens zweizähnig ist, wobei mindestens eine Koordinationsstelle von L¹ ein Stickstoffatom ist, zur Herstellung von Alkoholen der allgemeinen Formel (I) 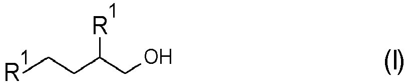 wobei die Gruppen R¹ verschieden sind oder gleich und gewählt aus C₂-C₃-Alkyl, linear oder verzweigt, unter Verwendung von mindestens einem Alkohol der Formel (II)
R¹-CH₂-CH₂-OH (II).

15. Verwendung nach Anspruch 14, **dadurch gekennzeichnet, dass** die Ru(II)-haltige Komplexverbindung mindestens einen weiteren Liganden aufweist, gewählt aus CO, Pseudohalogeniden, organischen Carbonylverbindungen, Aromaten, Olefinen, Phosphanen, Hydrid und Halogeniden.

16. Verwendung nach Anspruch 14 oder 15, **dadurch gekennzeichnet, dass** man L¹ wählt aus zweizähnigen und dreizähnigen Liganden, die über Stickstoffatome und gegebenenfalls über ein oder mehr Carben-Kohlenstoffatome mit Ru(II) koordinieren.

## Claims

1. A process for preparing branched alcohols of the general formula (I) 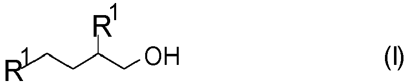 where the groups R¹ are different or identical and selected from C₂-C₃-alkyl, linear or branched, using at least one alcohol of the formula (II)
R¹-CH₂-CH₂-OH (II)
in homogeneous phase
in the presence of at least one base,
wherein at least one Ru(II)-containing complex compound is used in which the Ru(II) has at least one ligand L¹ which is at least bidentate, where at least one coordination site of L¹ is a nitrogen atom.

2. The process according to claim 1, wherein it is carried out without adding solvent which is different from alcohol of the formula (II).

3. The process according to claim 1 or 2, wherein it is carried out at temperatures in the range from 100 to 200°C.

4. The process according to any one of claims 1 to 3, wherein R¹ is selected from ethyl and isopropyl.

5. The process according to any one of claims 1 to 4, wherein L¹ is selected from bidentate and tridentate ligands which coordinate with Ru(II) via one or more nitrogen atoms and optionally one or more carbene carbon atoms.

6. The process according to any one of claims 1 to 5, wherein Ru(II)-containing complex compound has at least one further ligand selected from CO, pseudohalides, organic carbonyl compounds, aromatics, olefins, phosphanes, hydride and halides.

7. The process according to any one of claims 1 to 6, wherein L¹ is selected from compounds of the general formula (III) 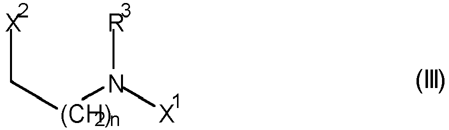 where the variables are selected as follows:
R³ is selected from hydrogen and C₁-C₅-alkyl,
n is selected from zero and one,
X¹ is selected from hydrogen, C₁-C₅-alkyl and (CH₂)ₙ₊₁-X²,
X² is selected from NR⁴R⁵, 2-pyridyl and imidazol-2-ylidenyl of the formula 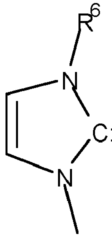
R⁴, R⁵ are identical or different and selected from C₁-C₁₀-alkyl, C₃-C₁₀-cycloalkyl, benzyl and phenyl, unsubstituted or mono- or polysubstituted with C₁-C₃-alkyl,
R⁶ is selected from C₁-C₁₀-alkyl, C₃-C₁₀-cycloalkyl, benzyl and phenyl, unsubstituted or mono- or polysubstituted with C₁-C₃-alkyl.

8. The process according to any one of claims 1 to 6, wherein L¹ is selected from compounds of the general formula (IV) 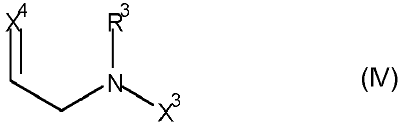 where the variables are selected as follows:
R³ is selected from hydrogen, C₁-C₁₀-alkyl, C₃-C₁₀-cycloalkyl, benzyl and phenyl,
X³ is selected from hydrogen C₁-C₅-alkyl and CH₂=X⁴,
X⁴ is selected from NR⁴, and
R⁴ is selected from C₁-C₁₀-alkyl and C₃-C₁₀-cycloalkyl, benzyl and phenyl, unsubstituted or mono- or polysubstituted C₁-C₃-alkyl.

9. The process according to any one of claims 1 to 6, wherein L¹ is selected from compounds of the general formula (V) 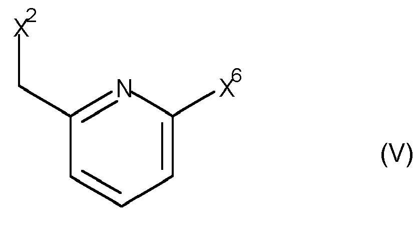 where the variables are selected as follows:
X⁶ is selected from hydrogen, C₁-C₅-alkyl and CH₂-X²,
X² is selected from NR⁴R⁵, 2-pyridyl and imidazol-2-ylidenyl of the formula 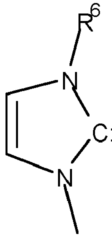
R⁴, R⁵ are identical or different and selected from C₁-C₁₀-alkyl, C₃-C₁₀-cycloalkyl, benzyl and phenyl, unsubstituted or mono- or polysubstituted with C₁-C₃-alkyl,
R⁶ is selected from C₁-C₁₀-alkyl, C₃-C₁₀-cycloalkyl, benzyl and phenyl, unsubstituted or mono- or polysubstituted with C₁-C₃-alkyl.

10. The process according to any one of claims 1 to 6, wherein L¹ is selected from compounds of the general formula (VI) 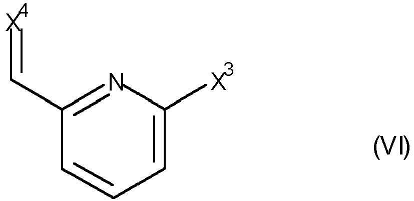 where the variables are selected as follows:
X³ is selected from hydrogen, C₁-C₅-alkyl and CH₂=X⁴,
X⁴ is different or identical and selected from N-R⁴,
R⁴ is selected from C₁-C₁₀-alkyl, C₃-C₈-cycloalkyl, benzyl and phenyl, unsubstituted or mono- or polysubstituted with C₁-C₃-alkyl.

11. The process according to any one of claims 1 to 10, wherein L¹ is selected from compounds of the formula (VII) 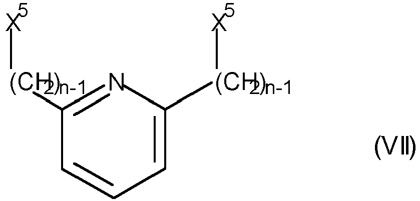 where the variables are selected as follows:
n is identical or different and in each case zero or one,
X⁵ is in each case identical and selected from NR⁴R⁵, 2-pyridyl and imidazol-2-ylidenyl of the formula 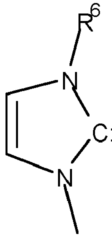
R⁴, R⁵ are different or identical and selected from C₁-C₁₀-alkyl, C₃-C₁₀-cycloalkyl, benzyl and phenyl, unsubstituted or mono- or polysubstituted with C₁-C₃-alkyl,
R⁶ is selected from C₁-C₁₀-alkyl, C₃-C₁₀-cycloalkyl, benzyl and phenyl, unsubstituted or mono- or polysubstituted with C₁-C₃-alkyl.

12. The process according to any one of claims 1 to 11, wherein the catalyst is produced in situ.

13. The process according to any one of claims 1 to 12, wherein alcohol of the formula (II) is used as azeotropic entrainer.

14. The use of catalyst comprising at least one Ru(II)-containing complex compound in which the Ru(II) has at least one ligand L¹ which is at least bidentate, where at least one coordination site of L¹ is a nitrogen atom, for preparing alcohols of the general formula (I) 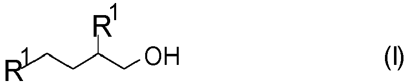 where the groups R¹ are different or identical and selected from C₂-C₃-alkyl, linear or branched, using at least one alcohol of the formula (II)
R¹-CH₂-CH₂-OH (II).

15. The use according to claim 14, wherein the Ru(II)-containing complex compound has at least one further ligand selected from CO, pseudohalides, organic carbonyl compounds, aromatics, olefins, phosphanes, hydride and halides.

16. The use according to claim 14 or 15, wherein L¹ is selected from bidentate and tridentate ligands which coordinate with Ru(II) via nitrogen atoms and optionally via one or more carbene carbon atoms.

## Revendications

1. Procédé de fabrication d'alcools ramifiés de formule générale (I) 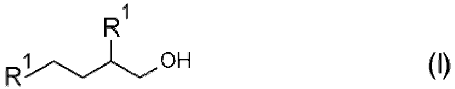 dans laquelle les groupes R¹ sont identiques ou différents, et choisis parmi alkyle en C₂-C₃, linéaire ou ramifié, en utilisant au moins un alcool de formule (II)
R¹-CH₂-CH₂-OH (II)
en phase homogène,
en présence d'au moins une base,
**caractérisé en ce qu'**au moins un composé complexe contenant Ru(II) est utilisé, dans lequel le Ru(II) comprend au moins un ligand L¹, qui est au moins bidentate, au moins un emplacement de coordination de L¹ étant un atome d'azote.

2. Procédé selon la revendication 1, **caractérisé en ce qu'**il est réalisé sans ajout d'un solvant différent de l'alcool de formule (II).

3. Procédé selon la revendication 1 ou 2, **caractérisé en ce qu'**il est réalisé à des températures dans la plage allant de 100 à 200 °C.

4. Procédé selon l'une quelconque des revendications 1 à 3, **caractérisé en ce que** R¹ est choisi parmi éthyle et isopropyle.

5. Procédé selon l'une quelconque des revendications 1 à 4, **caractérisé en ce que** L¹ est choisi parmi les ligands bidentates et tridentates, qui sont coordonnés par un ou plusieurs atomes d'azote et éventuellement un ou plusieurs atomes de carbone de carbène avec Ru(II).

6. Procédé selon l'une quelconque des revendications 1 à 5, **caractérisé en ce que** le composé complexe contenant Ru(II) comprend au moins un ligand supplémentaire, choisi parmi CO, les pseudohalogénures, les composés de carbonyle organiques, les composés aromatiques, les oléfines, les phosphanes, les hydrures et les halogénures.

7. Procédé selon l'une quelconque des revendications 1 à 6, **caractérisé en ce que** L¹ est choisi parmi les composés de formule générale (III) 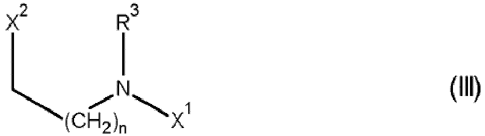 dans laquelle les variables sont choisies de la manière suivante :
R³ est choisi parmi hydrogène et alkyle en C₁-C₅,
n est choisi parmi zéro et un,
X¹ est choisi parmi hydrogène, alkyle en C₁-C₅ et (CH₂)ₙ₊₁-X²,
X² est choisi parmi NR⁴R⁵, 2-pyridyle et imidazol-2-ylidényle de formule 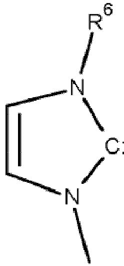
R⁴, R⁵ sont identiques ou différents, et choisis parmi alkyle en C₁-C₁₀, cycloalkyle en C₃-C₁₀, benzyle et phényle, non substitué ou substitué une ou plusieurs fois avec alkyle en C₁-C₃,
R⁶ est choisi parmi alkyle en C₁-C₁₀, cycloalkyle en C₃-C₁₀, benzyle et phényle, non substitué ou substitué une ou plusieurs fois avec alkyle en C₁-C₃.

8. Procédé selon l'une quelconque des revendications 1 à 6, **caractérisé en ce que** L¹ est choisi parmi les composés de formule générale (IV) 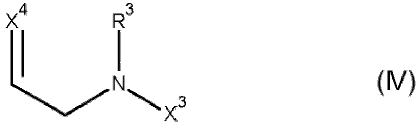 dans laquelle les variables sont choisies de la manière suivante :
R³ est choisi parmi hydrogène, alkyle en C₁-C₁₀, cycloalkyle en C₃-C₁₀, benzyle et phényle,
X³ est choisi parmi hydrogène, alkyle en C₁-C₅ et CH₂=X⁴,
X⁴ est choisi parmi NR⁴, et
R⁴ est choisi parmi alkyle en C₁-C₁₀ et cycloalkyle en C₃-C₁₀, benzyle et phényle, non substitué ou substitué une ou plusieurs fois avec alkyle en C₁-C₃.

9. Procédé selon l'une quelconque des revendications 1 à 6, **caractérisé en ce que** L¹ est choisi parmi les composés de formule générale (V) 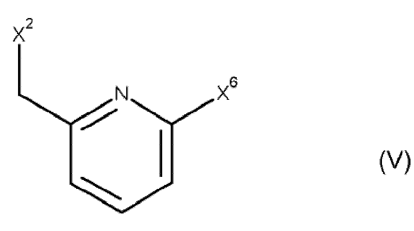 dans laquelle les variables sont choisies de la manière suivante :
X⁶ est choisi parmi hydrogène, alkyle en C₁-C₅ et CH₂-X² ,
X² est choisi parmi NR⁴R⁵, 2-pyridyle et imidazol-2-ylidényle de formule 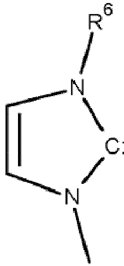
R⁴, R⁵ sont identiques ou différents, et choisis parmi alkyle en C₁-C₁₀, cycloalkyle en C₃-C₁₀, benzyle et phényle, non substitué ou substitué une ou plusieurs fois avec alkyle en C₁-C₃,
R⁶ est choisi parmi alkyle en C₁-C₁₀, cycloalkyle en C₃-C₁₀, benzyle et phényle, non substitué ou substitué une ou plusieurs fois avec alkyle en C₁-C₃.

10. Procédé selon l'une quelconque des revendications 1 à 6, **caractérisé en ce que** L¹ est choisi parmi les composés de formule générale (VI) 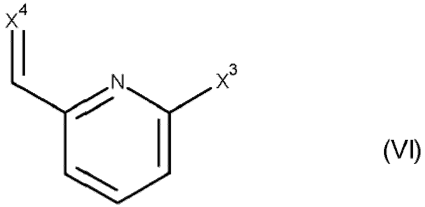 dans laquelle les variables sont choisies de la manière suivante :
X³ est choisi parmi hydrogène, alkyle en C₁-C₅ et CH₂=X⁴,
les X⁴ sont identiques ou différents, et choisis parmi N-R⁴,
R⁴ est choisi parmi alkyle en C₁-C₁₀, cycloalkyle en C₃-C₈, benzyle et phényle, non substitué ou substitué une ou plusieurs fois avec alkyle en C₁-C₃.

11. Procédé selon l'une quelconque des revendications 1 à 10, **caractérisé en ce que** L¹ est choisi parmi les composés de formule générale (VII) 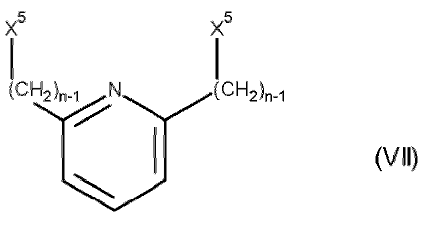 dans laquelle les variables sont choisies de la manière suivante :
les n sont identiques ou différents, et représentent à chaque fois zéro ou un,
les X⁵ sont à chaque fois identiques et choisis parmi NR⁴R⁵, 2-pyridyle et imidazol-2-ylidényle de formule 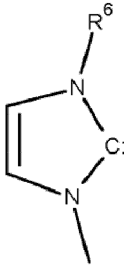
R⁴, R⁵ sont identiques ou différents, et choisis parmi alkyle en C₁-C₁₀, cycloalkyle en C₃-C₁₀, benzyle et phényle, non substitué ou substitué une ou plusieurs fois avec alkyle en C₁-C₃,
R⁶ est choisi parmi alkyle en C₁-C₁₀, cycloalkyle en C₃-C₁₀, benzyle et phényle, non substitué ou substitué une ou plusieurs fois avec alkyle en C₁-C₃.

12. Procédé selon l'une quelconque des revendications 1 à 11, **caractérisé en ce que** le catalyseur est formé in situ.

13. Procédé selon l'une quelconque des revendications 1 à 12, **caractérisé en ce que** l'alcool de formule (II) est utilisé sous la forme d'un agent d'entraînement azéotropique.

14. Utilisation de catalyseurs, contenant au moins un composé complexe contenant Ru(II), dans lequel le Ru(II) comprend au moins un ligand L¹, qui est au moins bidentate, au moins un emplacement de coordination de L¹ étant un atome d'azote, pour la fabrication d'alcools de formule générale (I) 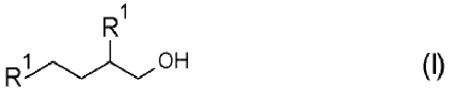 dans laquelle les groupes R¹ sont identiques ou différents, et choisis parmi alkyle en C₂-C₃, linéaire ou ramifié, en utilisant au moins un alcool de formule (II)
R¹-CH₂-CH₂-OH (II) .

15. Utilisation selon la revendication 14, **caractérisée en ce que** le composé complexe contenant Ru(II) comprend au moins un ligand supplémentaire, choisi parmi CO, les pseudohalogénures, les composés de carbonyle organiques, les composés aromatiques, les oléfines, les phosphanes, les hydrures et les halogénures.

16. Utilisation selon la revendication 14 ou 15, **caractérisée en ce que** L¹ est choisi parmi les ligands bidentates et tridentates, qui sont coordonnés par des atomes d'azote et éventuellement par un ou plusieurs atomes de carbone de carbène avec Ru(II).
